# EUROPEAN PATENT SPECIFICATION

(11) **EP 1 931 463 B1**
(45) Date of publication and mention of the grant of the patent: **22.04.2009**
(21) Application number: 06792093.4
(22) Date of filing: 15.09.2006
(51) Int. Cl.: B01J 19/18, B01D 11/04, C12P 7/18, C12P 7/62, C12P 13/00

(54) **CONTINUOUS CHEMICAL PROCESSES IN CENTRIFUGAL CONTACT SEPARATORS**
KONTINUIERLICHE CHEMISCHE VERFAHREN IN ZENTRIFUGALKONTAKTSEPARATOREN
PROCEDES CHIMIQUES CONTINUS DANS DES CONTACTEURS-SEPARATEURS CENTRIFUGES

(30) Priority: 15.09.2005 EP 05077102
(43) Date of publication of application: 18.06.2008
(73) Proprietor: DSM IP Assets B.V., 6411 TE Heerlen (NL)
(72) Inventor: VRIES, DE, Johannes, Gerardus, (Hans), NL-6228 GZ Maastricht (NL); KWANT, Gerard, Jan, NL-6137 LM Sittard (NL); HEERES, Hero, Jan, NL-9617 EM Harkstede (NL)
(74) Representative: Hoogendam, Gerrie Christine
(86) International application number: PCT/EP2006/009012
(87) International publication number: WO 2007/031332

(56) References cited:
- GB-A- 1 223 610
- GB-A- 1 502 173
- US-A- 2 106 521
- US-A- 2 995 612
- US-A- 4 017 263
- TAYLOR R J ET AL: "The applications of formo- and aceto-hydroxamic acids in nuclear fuel reprocessing" JOURNAL OF ALLOYS AND COMPOUNDS, ELSEVIER SEQUOIA, LAUSANNE, CH, vol. 271-273, 12 June 1998 (1998-06-12), pages 534-537, XP004181845 ISSN: 0925-8388

## Description

The invention relates to the use of a centrifugal contact-separator for carrying out a reaction. The invention also relates to a process for carrying out a reaction in a centrifugal contact-separator, and to a process for carrying out a catalytic reaction in a centrifugal contact-separator.

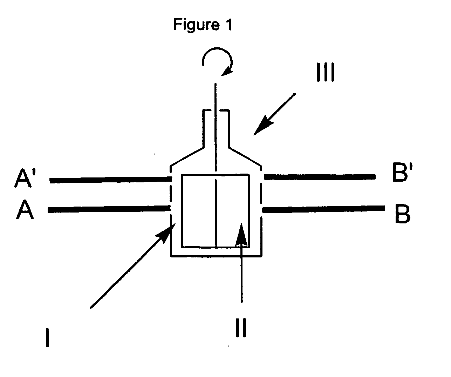

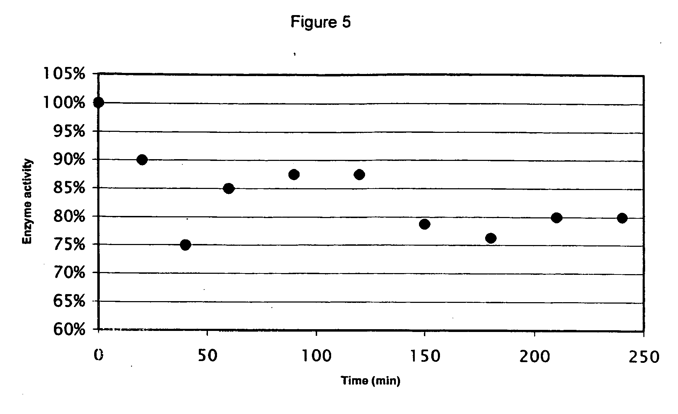

Centrifugal contact-separators are known devices. In Fig. 1/5 a cross-section of such a centrifugal contact separator is shown. They typically comprise an inner cylinder rotatably disposed in an outer cylindrical housing. Typically, two inlets are connected to the outer housing; one for each of two immiscible phases, A and B respectively, that are pumped into the device. In the centrifugal contact separator the two liquid phases are mixed intimately (See Fig. 1/5 for a schematic representation) by the rotating force of the inner cylinder. The mixing occurs on the outside of the inner cylinder and to some extent probably also in the inner cylinder where the mixture enters the inner cylinder. Subsequently separation of the two phases takes place in the inner cylinder through the centrifugal force exerted by the rapidly rotating inner cylinder. After separation, two phases A' and B', respectively, each leave the device through two separate outlets (Fig. 1/5).

The known use of centrifugal contact-separator devices having an inner cylinder rotatably disposed in an outer cylinder housing, is the separation of mixtures of liquids, based on the differences in the densities of these liquids. For example, US 3,931,928 describes a mixed liquid that is introduced into the inner cylinder and the latter has a concentric opening at one longitudinal end thereof through which separated components of lesser specific gravity pass. The use of contact-separators is known in the field of radioactive materials processing. Taylor et al. (1998) [Journal of Alloys and Compounds 271-273, 534 - 537] describe the separation of Pu(IV) and Np(IV) from U(VI) which is relevant in nuclear fuel reprocessing, whereby the use of centrifugal contactors in studying the separation of complexes of Np and U is suggested. A further known use is the reaction of gases with liquids, or with liquid or solid substances in finely divided form in liquids in reaction centrifuges reported in GB 1,223,610.

US 2,995,612 discloses the use of a centrifugal contactor containing a number of revolving concentric cylinders in catalytic alkylation reactions of alkanes, aromatics or naphthenes in the presence of liquid catalysts wherein a film-type contact between the catalyst and the hydrocarbon phases is achieved, thus sharply limiting the contacting of the alkylated products with the catalyst in order to prevent side reactions.

Many other centrifugal contact-separator devices have been described, such as, for example, the CINC separators available from Costner Industries Nevada Corporation (CINC). Separators of this type are generally known and are described in, for example, the following patents: U.S. Patent Nos. 3,931,928, 3,955,757, 4,175,040, 4,525,155, 4,634,416, 4,816,152, 4,959,158, 5,024,647, 5,762,800, 6,346,069, 6,607,473, 6,719,681. Additional information may be found, for example, at the website for Costner Industries at www.cit-ind.com. Additional related information may be found at the web pages of Auxill Nederland BV, a distributor of the CINC separators (also referred to as CIT liquid-liquid centrifuges) (see www.auxill.nl/uk/cit.separators.php).

In addition to the CINC centrifugal contact-separators, centrifugal separators or extractors are also available from Rousselet et Robatel of Annonay, France, such as, for example, such as the Centrifugal Separator, Model 8XP or Model BXP 360P. Other sources of centrifugal extractors will be well known to the practitioner.

These centrifugal separators may be used in any of the sizes available and in any of the forms offered by their vendors. For example, models of the CINC centrifugal contact-separators come in sizes ranging from 0.5 gal. per min. (1.9 liter/min) (Model V-02) to 600 gal/min (2271 I/m) (Model V-36) with intermediate ranges of 6 gpm (22.7 Ipm) (Model V-05), 30 gpm (113.5 Ipm) (Model V-1 0), 90 gpm (340.6 Ipm) (Model V16) and 200 gpm (757 Ipm) (Model V-20).

A problem in the known processes for carrying out chemical reactions, and in particular for fast and/or exothermic reactions, is that due to mass and/or heat transfer problems, local concentration and/or temperature gradients occur in the equipment so far used for carrying out reactions, resulting in a possible runaway of the reaction that is carried out. It is also possible, that by simply contacting reactants an explosive mixture is formed. In addition, the selectivity of the reaction may suffer from local high gradients of reactants or from local hot spots. In the production of fine chemicals multi-purpose batch reactors are almost always used for chemical reactions. It is obvious that batch reactors are not very suitable to tackle problems related to mass and heat transfer. Excellent continuous reactors are known, but their design makes them usually suitable for only a single process, such as in bulk chemical processes. These solutions are thus not suitable for the production of fine chemicals. Known measures to prevent the occurrence of uncontrollable mass and/or heat transfer problems in fine chemicals production are for instance micro reactors in which the reactants are pumped through channels with a diameter of just a few micrometers, which allows very rapid mixing and dissipation of heat. However, these micro reactors are relatively expensive and are scaled up by using parallel set ups of many micro reactors. In this way no scale advantage is obtained. In addition, the small channels are prone to blockage through fouling, either through small particulates or by solids that are formed as by-product in the reaction. Thus, there is a need for a device, which allows fast reactions to take place, which almost instantaneous intimate mixing of the reactants and that can be scaled up in the usual manner by increasing its size. In addition the device should be flexible enough to be used as multi-product equipment.

Surprisingly, it has now been found that it is possible to carry out reactions in a safer way, by conducting them in a two-phase system in a continuous or semi-continuous manner in a centrifugal contact-separator. Thus, the invention relates to the use of a centrifugal contact-separator for carrying out a reaction in a liquid-liquid emulsion formed from two immiscible liquids, wherein the reaction is carried out between at least two reactants. The invention also relates to the use of a centrifugal contact-separator as described above, wherein the reaction is a catalytic reaction and wherein the catalyst may be a homogeneous catalyst, which may be an enzyme or a transition metal catalyst. The invention also relates to the use of a centrifugal-contact separator for carrying out a kineticresolution.

The reaction is a non-radioactive reaction, which in the context of this invention is defined as any reaction not involving any compound comprising at least one of the atoms U, Np or P.

An emulsion is a mixture of two immiscible liquids. One of the two liquids is dispersed in the form of tiny droplets in the other liquid. The phase formed by the droplets is often called the disperse phase and the other phase is called the continuous phase.

For the purpose of this invention, a reaction is defined as a process in which the desired end product is formed in the centrifugal contact separator; i.e. the desired product is not introduced at the start of the process, as may for example be the case in pure separation reactions.

In a centrifugal contact-separator that is used for a continuous reaction, the volume of the reaction zone is relatively small compared to the total volume of reactants to be reacted and/or products. In case a reaction is carried out using reactants that either may react in a violent manner, or are prone to runaways or that may form an explosive mixture or explosive side-products, the effects of an explosion, if it would occur, would be much smaller than they would be using the same volume of reactants in a batch reactor. Moreover, the mixing in a contact-separator is very fast, and the small volume and the fast mixing both contribute to the prevention of uncontrollable mass or heat transfer problems and thus, runaways are less likely to occur. In addition, the residence time can be adjusted to prevent excessive contact between the reactants that could possibly lead to the formation of explosive side products.

Although the reaction volume of centrifugal contact separators is relatively small with respect to the total volume of reactants to be reacted, the use of a centrifugal contact-separator still allows good yields because of the relatively high flows that are possible.

Thus, the invention relates to a continuous process for carrying out a reaction, comprising the steps of:
i) continuously introducing a liquid phase (A) and a liquid phase (B) into at least one first centrifugal contact-separator, wherein liquid phases (A) and
   (B) are immiscible and wherein phase (A) and/or phase (B) comprise at least one reactant,
ii) continuously mixing phase (A) and phase (B) thereby allowing an emulsion to be formed
iii) applying a centrifugal force that allows phase separation of the emulsion, such that phases (A') and (B') are obtained,
iv) optionally, recovering a product

Preferably, more than 70%, more preferably at least 90% and most preferred more than 90% of the total amount of product that has been produced by the reaction in the centrifugal contact-separator is present in either liquid phase (A') or in liquid phase (B'), and, if the product is recovered, that percentage is preferably recovered in step iv. The product is recovered from the phase wherein preferably more than 70%, at least 90% and most preferred more than 90% of the total amount of the product is present.

Many variations are possible in this process. The continuous process can be used to carry out different types of reactions, e.g. decompositions reactions , whereby only phase A comprises a reactant and whereby after the reaction has taken place at least one of the decomposition products is predominantly present in either phase A' or B' and the other decomposition product preferably is predominantly present in the other phase, thus allowing easy recovery of each of the products. With the term predominantly present is meant that more than 50% of the total amount of a compound is present in one of the phases. This process may be highly beneficial in reactions in which the equilibrium of the reaction is on the side of the reactant. Continuously removing the product will allow the reaction to proceed beyond the equilibrium amount.

Thus, in an embodiment the invention relates to a continuous process for carrying out a reaction, comprising:
I. continuously introducing
   i. a liquid phase (A) comprising a reactant and
   ii. a liquid phase (B),
   and which phases (A) and (B) are immiscible, into at least one first centrifugal contact-separator;
II. continuously contacting the liquid phase (A) and the liquid phase (B) in the at least one first centrifugal contact-separator, whereby upon contact of the two phases a mixture is formed and a reaction takes place resulting in a first and a second products and whereby due to differences in density the mixture in the contact-separator separates into phase (A') and phase (B') and wherein after separation of the two phases (A') and (B') at least 90 % of the total amount of the first product that has been produced in the contact-separator is present in either liquid phase (A') or in liquid phase (B') and whereby at least 90 % of the total amount of the second product that has been produced is present in the other phase.
III. after step II, optionally recovering the first product and/or the second product from the phase wherein at least 90% of the total amount of that product is present.

In the frame-work of this invention, a continuous process is defined as any process that is not a pure batch process. A pure batch process is defined as a process wherein after a first amount of each necessary reactant has been introduced and the reaction has started no additional reactant is added to the reaction mixture.

Phases A and B are defined to be immiscible when after introduction of phase A and phase B into the centrifugal contact-separator without any reactant or catalyst or anything else added to them, but otherwise subjected to the same process condition as during reaction, the resulting phase A' obtained at the outlet of the centrifugal contact-separator contains no more than 5 vol% of B, preferably no more than 2 vol%, more preferably no more than 1, and most preferably no more than 0.2 vol% of Phase B. The term density is used for the mass of phase A, phase B, phase A' or phase B', respectively, per unit volume.

It will be understood that A is used to describe phase A at the reactor inlet. At the outlet, the composition of phase A will typically be somewhat different, as a result of passing through the centrifugal contact separator with phase B. Thus, the composition at the outlet that has a similar but not the same composition as phase A, is indicated as being phase A'. The same applies to phase B, which at the outlet is denoted as phase B'.

Typically, the first reactant used in the process according to the invention is an organic compound. The second reactant which may be present typically may be an organic or inorganic compound.

The process according to the invention, wherein a centrifugal contact-separator is used to carry out a reaction, is preferably a process wherein a homogeneous catalyst is used. By carefully selecting the solvents used in phase A and phase B, use of the centrifugal contact-separator makes it possible to end up with a phase A' containing the homogeneous catalyst and a phase B' containing the desired product, or vice-versa, and, moreover, use of a centrifugal contact-separator makes it possible to carry out a homogenous catalytic reaction is a continuous process.

In the chemical industry there is a need for kg scale to ton scale production facilities that allow efficient production of various products in the same facility. This is for example the case in the so-called custom-manufacturing industry, which supplies the pharmaceutical and agro industries with intermediates. In custom manufacturing, the amount of a certain product that is required is so large that it can no longer efficiently be produced on lab scale, but it is not so large that a dedicated plant can be build for it. So far, this problem is typically solved by batch-wise production of the desired product in multi-purpose equipment. Very often, a number of the necessary reactions to synthesize such a product, are catalytic reactions. In particular catalytic reactions carried out with a homogeneous catalyst such as a homogeneous transition metal catalyst, a biocatalyst, which may for instance be an enzyme or whole cells containing catalytically active enzymes, an organocatalyst or an acidic or basic catalyst are often required in the synthesis of the desired product.

It is known to carry out batch wise reactions using a homogeneous catalyst in a two-phase liquid system, wherein one phase contains the homogeneous catalyst and the other phase contains the reactant which will be catalytically converted to the product, and which remains in this same phase. Such a process comprises the following steps:
- filling of the tank with the reactant phase
- addition of the catalyst phase to start the reaction while mixing to obtain an emulsion of the two phases
- reacting until final conversion
- optionally cooling or heating the phases in the reactor during reaction
- settling of the two phases
- removal of one phase from the reactor
- removal of the second phase from the reactor
- cleaning of the reactor
- returning the catalyst phase to the reactor so the next batch may be started.

In a multi-product batch plant reaction time and settling time may vary considerably, dependent on the reaction system. To reach sufficient settling, a very long time may be needed which may cause the product to react further to undesired by-products. Other problems, associated with carrying out two-phase catalytic reactions in a batch reactor are:
1. When reactions are fast the product may react away because the residence time is unavoidably too long.
2. When reactions are fast it may be troublesome to remove the heat of reaction and side-products may be formed because of the high temperatures.
3. When reactions are fast the catalyst may deactivate as it is in an active state and for lack of substrate to react which, it will engage in unwanted reactions that eventually lead to its destruction. The same fate may befall the catalyst during the separation of the layers and thereafter.
4. Even if the catalyst is stable it may deactivate because traces of oxygen come into the reactor during the draining of the catalyst and/or product phase.

It is clear from the above that it is highly desirable to keep the catalyst inside the reactor and to make sure that always at least some of the reactants are present to prevent its deactivation. Thus it would be highly desirable to carry out these catalytic reactions in a continuous mode.

Continuous processes for carrying out two-phase reactions using a homogeneous catalyst are known, but only for bulk products, where the size of the product, typically in the order of 100 000 tons/y, justifies the investment in a dedicated continuous plant. In these processes, the equipment used and the connection between different pieces of equipment is designed for the use in one particular reaction with particular starting materials and for recovering a particular product. In such a situation, use of a continuous process wherein a homogenous catalyst is used is economically feasible. However, in the fine chemicals industry or the so-called custom manufacturing industry, there is a need for replacing batch processes that are typically used when only a relatively small amount of product is required by a customer, by continuous processes, without giving up the flexibility that batch process equipment provides. For batch processes it is simple to use the same reactor every time for a different reaction, since recovery of the product and (optionally) catalyst take place "off line". When not enough product can be made in one batch, the use of batch processes is clearly less attractive, because every batch may result in a different quality of the product, and time is lost emptying the batch reactor and starting a new batch.

Surprisingly, it has now been found that the use of a centrifugal contact-separator provides a solution to the above-mentioned problems related to the custom-manufacturing industry. Centrifugal contact separators are relatively cheap pieces of equipment and require relatively little space. Use of a centrifugal-contact separator allows the continuous production of desired products, also when the reaction to be carried out is a reaction carried out in the presence of a homogenous catalyst.

Therefore, in an embodiment the invention relates to a continuous process for carrying out a reaction with a homogenous catalyst, comprising:
I. continuously introducing
   a liquid phase (A) comprising the homogeneous catalyst and optionally comprising a reactant, and a liquid phase (B) optionally comprising a reactant, into at least one first centrifugal contact-separator; and wherein phases (A) and (B) are immiscible
II. continuously contacting the liquid phase (A) and the liquid phase (B) in the at least one first centrifugal contact-separator, whereby upon contact of the two phases a mixture is formed wherein the catalyst is contacted with one or more reactants and a catalytic reaction takes place resulting in at least a first product and whereby due to differences in density the mixture in the contact-separator separates into phase (A') and phase (B') and whereby after separation of the two phases (A') and (B') the catalysts is mostly present in liquid phase (A') and the product is mostly present in phase (B');
III. after step II, optionally, continuously recirculating phase (A') and/or optionally regenerating the catalyst and/or optionally removing a by-product (from phase (A'))
IV. after step II, optionally recovering the product from phase (B')

In the framework of this invention, a homogenous catalyst is defined as any catalyst that is present in the same physical state as the reactants. Typically, the reactants are dissolved, and thus, typically the catalyst is also dissolved. In the framework of this invention, the term homogeneous catalyst covers different types of catalysts including those described above that will typically be dissolved in the aqueous phase (A or A'). Enzymes and whole or disrupted cells are not always fully dissolved, but nonetheless can be used in the process according to the invention in the same way as dissolved catalysts. Thus, the process according to the invention explicitly covers the use of enzymes or whole cells as homogenous catalysts. Also, catalysts that are in a gel-like or in a colloidal state are considered to be dissolved, for the purpose of this invention. Although in theory all materials dissolve to some extent, in the process according to the invention, a catalyst counts as dissolved if it is present in solution in an amount high enough to catalyse the desired reaction and provided that the amount of non-dissolved catalyst is not so high that it causes problem in carrying out the continuous process. Preferably, more than 50wt% of the total amount of a catalyst is present in the centrifugal contact-separator in the dissolved form, more preferably 70%, most preferably the catalyst is completely in solution. These latter preferred embodiments do not apply to colloids, gels, enzymes and whole cells, which, as described above, are considered homogenous catalysts for the purpose of this invention.

With the expression "mostly present" in connection with the catalyst is meant that more than 70% of the catalyst, preferably more than 90%, more preferably more than 95, and most preferably more than 99%, of the catalyst after separation of the two phases is present in phase A'. It should be noted that when not all of the catalyst present in the centrifugal contact separator is dissolved, the percentages apply only to the amount of the catalyst that is dissolved.

Phase transfer catalysts or co-catalysts, if present, may not be included when determining the amount of catalyst present in phase A'.

The expression "mostly present" in connection with the product means that more than 70% of the product, preferably more than 90%, more preferably more than 95, and most preferably more than 99%, of the product after separation of the two phases is present in phase B'.

The processes according to the invention are carried out in two immiscible phases A and B. The liquid or solvent(s) forming phase A can for example be water or an aqueous solution and the liquid or solvent(s) forming phase B can in that case for example be an organic solvent or a mixture of organic solvents. When phase A consists of an aqueous solution all organic solvents that are substantially immiscible with water can be used to form phase B, for example: non polar solvents such as hexanes, heptanes petroleum ether, aromatic solvents such as toluene and xylene, halogenated solvents, such as dichloromethane, dichloroethane or chlorobenzene, polar solvents such as MTBE, valerolactone, ethyl acetate, butyl acetate, higher alcohols such as octanol and the like. It is also possible that the reactant(s) and/or the product of the reaction form an organic phase, which can than be seen as phase B or as forming part of phase B.

Typically, phase B will comprise an organic solvent and a reactant(s). In case phase B is an organic phase, the concentration of the reactant(s) is limited only by their solubility in phase B; the concentration of the product is limited to its solubility in phase B'. It is clear that for an economic process the concentration should be as high as possible, however, in some cases it may be more beneficial to work at lower concentrations, for instance concentrations between 1-10 mol%. This may be for reasons of selectivity or for safety reasons.

The phases A and B may contain one or more additional reactants. Usually the reactant(s) will be present in solution in substantially the same molar concentration, although in some cases it can be beneficial to use different molar concentration of the various reactants.

Either phase A or B or both may also contain additives that have a positive effect on the reaction, such as surface-active compounds, for instance sodium dodecylsulfonate and phase transfer catalysts, for instance tetra-alkyl ammonium salts. Examples are tetrabutylammonium bromide and methyltrioctylammonikum hydrogensulfate.

When phase A is an aqueous solution, the aqueous phase A contains either a reactant, or a catalyst or both. It is also possible that the aqueous phase contains multiple reactants or catalysts. The concentration of the catalysts and reactants may vary but is limited by their solubility in the aqueous phase. The concentration of the catalyst is also determined by its reactivity: Preferably the concentration of the catalyst is chosen such that upon a single pass through the centrifugal contact separator a conversion of at least 20%, but preferably at least 50% is reached. The concentration of the reactant in the aqueous phase is kept at a high enough level to allow the desired conversion of the reactant in the organic phase as described above. The aqueous phase may be buffered in the usual manner to keep it at a desired pH value if necessary.

Preferably, the processes according to the invention are carried out with all components present in phase A and B, and after reaction in phase A' and B', either being liquid or being fully dissolved.

In some cases an organic reactant may be used that is water-soluble, In that case it may be beneficial to revert the above system. In this case a second reactant and/or the catalyst may be present in the organic phase.

In addition to the organic/aqueous systems described above it is also possible to use combinations of an organic solvent, or mixture thereof with an ionic liquid. Ionic liquids are described in P. Wasserscheid & T. Welton, Ionic Liquids in Synthesis, Wiley-VCH, Weinheim, 2002. The ionic liquid may contain varying amounts of water. The ionic liquid phase contains either a reactant, a catalyst or both. It is also possible that the ionic liquid phase contains multiple reactants or catalysts.

Another possible solvent combination for phases A and B is the use of two immiscible organic solvents that differ strongly in polarity. Examples of such combinations are ethylene glycol/hexane or butanediol/hexane. Other combinations are possible. The polar phase contains the catalyst and/or reactants, whereas the nonpolar phase contains the organic reactant, the product and possibly one or more reactants.

Yet another possible solvent combination for phases A and B, respectively is a combination of a fluorous solvent, such as perfluorohexane, perfluorooctane or perfluoromethylcyclohexane in combination with an organic solvent that is immiscible at ambient temperature with the fluorous solvent such as hexane, toluene, chlorobenzene, THF or isopropanol or mixtures thereof. In this case, the catalyst may be made soluble in the fluorous phase by attaching fluorocarbon groups to it.

Of course, any possible combination of phases A and B may be applied, as long as the phases are immiscible. It is also possible to use one of the reactants as the solvent.

Many types of reactions can be performed in the centrifugal contact separators. The set up is particularly useful for reactions that are highly exothermic or that contain hazardous substances. One possibility is the production of an azide by reaction between an azide salt, such as sodium azide in the aquous phase with an organic halide or an epoxide which is dissolved in the organic phase.

The invention is also particularly useful for reactions in which one or more reactants reacts with a mineral acid or base that is dissolved in the aqueous phase. Examples of mineral acids are sulfuric acid or hydrochloric acid. Examples of mineral bases are NaOH, KOH, NaHCO₃, Na₂CO₃, CsCO₃. An example of a base-catalysed process is the hydrolysis of an ester or an amide. Example of an acid catalysed process is the hydrolysis of an ester or an amide or the formation of an ester from an alcohol and a carboxylic acid.

Preferably, the catalysts used in the invention are catalysts that are soluble only in the phase that does not contain the substrate. However, exceptions are the use of phase transfer catalysts, which may be chiral, and the use of organocatalysts. In this case it may be advantageous to remove these catalysts from the solution containing the product after reaction has taken place by an extraction process for which another contact separator may be used.

The term substrate is used herein to indicate the reactant, that ultimately forms the larger part of the product that is produced in the contact separator. Many reactions are carried out between two reactants, one of which is a relatively large molecule and one of which is a relatively small molecule. In this patent, the larger of the different molecule is referred to as substrate.

The homogeneous catalysts used in the invention may be transition metal catalysts, based on complexes or clusters of transition metals of group 5-12 which complexes will have the general formula MₐLig*_{b}Lig_{c}I_{d} in which M is a transition metal of group 5-12, Lig* is a chiral ligand, which may be monodentate, bidentate, tridentate of tetradentate. Lig is a non-chiral ligand, I is a counterion, a is an integer from 1-18 and b,c,d are integers from 0-36. These catalysts may need special features to secure they are soluble in phase A and not in phase B (or vice versa). In the case the catalyst is in the aqueous phase it can be useful to attach water-soluble groups (such as CO₂Na, SO₃Na or PO₃K or oligoethyleneglycol units), in case of the above-mentioned complexes to either Lig*, Lig or I. These same features will probably also aid the dissolution if the polar phase is an ionic liquid. In case the catalyst phase is a fluorous solvent, the fluorocarbon groups will need to be attached to either Lig, Lig* or I.

Examples of metals are Rh, Ru, Pd, Ir, Pt, Cu, Au, Os, Co, Ni, Hf, Ta, Re, Mo, Mn; examples of chiral ligands Lig* are enantiopure bipyridines, diimines, oxazolines, bisoxazolines, phosphinooxazolines, bisphosphines, such as BINAP, DIOP, Josiphos, DUPHOS; bisphosphites, bisphosphonites, monodentate phosphorus ligands such as phosphines, phosphoramidites, phosphites or phosphonites. In case b is >1 the b Lig* groups need not be the same.

Examples of ligands Lig are phosphines, bisphosphines, aminophosphines, alkylamines, imines, pyridines, diamines, diimines, bipyridines, oxazolines, bisoxazolines, ethers, such as THF, solvents, such as acetonitrile, olefins, such as maleic anhydride, dienes such as cyclooctadiene or norbornadiene.

Examples of counterions I are chloride, bromide, fluoride, acetate, trifluoroactetate, BF₄·PF₆, tetraphenylborate or fluorinated forms thereof, such as BARF.

The catalysts used in the invention may also be transition metal based colloids. These colloids may carry chiral (Lig*) and non chiral ligands (Lig) or mixtures thereof. They may also be stabilized by salts, such as NaBr or tetraalkylammonium salts or by polymers such as poly-N-vinyl-pyrrolidinone. The catalysts of the invention may also be organocatalysts, which may or may not be chiral. Examples are derivatives of Cinchona alkaloids, polypeptides, diketopiperazines, such as cyclo-Phe-His, sugar derivatives such as Yian Shi's fructose-based ketone, proline and its derivatives, chiral dimethylaminopyridine derivatives, These catalysts may also carry groups that make them soluble in phase A as described above for the transition metal catalyst

The catalysts used in the invention may be an enzyme, such as an oxidoreductase, for instance Alcohol dehydrogenase; a transferase, such as Nicotinamide N-methyltransferase; a hydrolase such as Carboxylesterase; a lyase, such as Pyruvate decarboxylase; an isomerase/racemase such as Alanine racemase or a ligase, such as Tyrosine-tRNA ligase; hydrolytic enzymes such as a peptidase an esterase or a lipase . In principal, any enzyme displaying useful catalytic activity can be used in the present invention.

The catalysts used in the invention may also be whole cells, which may or may not be disrupted. These cells will contain active catalysts such as enzymes, which may be genetically altered and overexpressed if this is deemed necessary.

Reactions that are conveniently carried out according to the invention are for instance reactions in which the substrate is the reactant in the organic phase, which reacts with the reactant in the aqueous phase. An example could be the reaction of an organic halide or an epoxide as the organic reactant in phase B with sodium azide as inorganic reactant in aqueous phase A. By performing the reaction in this way the build-up of dangerous HN₃ can be avoided. Another possible reaction would be an elimination reaction in which water or a hydrogen halide is eliminated from the substrate (the organic reactant residing in phase B). As the side products (water or hydrogen halide) are continuously removed in phase A the equilibrium of these reactions will be driven to the desired side.

Oxidation reactions are also conveniently executed according to the invention. The substrate will typically be present in the organic phase (organic reactant in phase B) and reacts in the centrifugal contact-separator with the oxidant, which is dissolved in phase A. the oxidant could for instance be hydrogen peroxide or sodium hypochlorite. It is clear that if large amounts of an organic substrate would be in prolonged direct contact with a strong oxidant like these in a batch reactor, the danger of an explosion or a run-away reaction is very high. By executing the reaction in the centrifugal contact separator these risks are greatly reduced.

Catalytic reactions catalysed by transition metal complexes are also conveniently carried out according to the invention. An example would be the reduction of an aldehyde, a ketone or an imine, which is the organic reactant in phase B using a water-soluble ruthenium catalyst which is supplied in phase A which is either aqueous, an ionic liquid or fluorous. The reductant could be iso-propanol which could be present in phase B; it could also be a formiate salt which could be present as reactant in Phase A. Products would be primary alcohols, secondary alcohols or an amine, respectively. Water-soluble ruthenium catalysts are well-known, for instance from the work of J.M.J Williams (Tetrahedron Letters (2001), 42(24), 4037-4039).

Many other examples of two-phase aqueous catalysis are described in "Aqueous-Phase Organometallic Catalysis, Eds. B. Cronils and W.A. Herrmann, Wiley-VCH, Weinheim, 1998 and the second edition from 2004. In general all these reaction types can be advantageously performed in a centrifugal contact separator.

Many enzymatic processes can conveniently be carried out in the centrifugal contact separators. Examples are the hydrolysis of esters and amides. These can be conveniently carried out in aqueous/organic systems. It is also possible to do transesterification reactions. These reactions can be performed with the enzyme in the ionic liquid phase and the organic reactant in the organic solvent.

It is also highly advantageous to carry out a catalytic kinetic resolution in the contact separator. In these processes a catalyst will selectively react with only one enantiomer in a racemic mixture, leaving the other enantiomer largely untouched. Enzymatic kinetic resolution processes are well-known. One example is the use of a lipase for the resolution of an ester of a racemic secondary alcohol or th ester of a racemic carboxylic acid. By carrying out a lipase-catalysed hydrolysis reaction till between 50-60% conversion, one of the two enantiomers will be completely hydrolysed to the alcohol and the carboxylate, whereas the other enantiomer of the ster is retained in its enantiopure form. The process according to the present invention is highly suitable for carrying out these catalytic resolution reactions. If these reactions are carried out in batch it is always hard to determine the exact point where the reaction should be stopped unless some in-situ real time monitoring is done. In the equipment of the present invention the parameters (flow rates, rotation speed, catalyst concentration, temperature) of the reaction can be manipulated in such a way that conversion is maintained at exactly the desired percentage.

Although not described in detail herein, it will be understood that it is possible to use more centrifugal contact -separator in series to achieve an optimum process. This will allow a series of reactions to be performed consecutively.

The invention covers many different embodiments of continuous processes in centrifugal contact-separators, e.g. phase A comprises a reactant and phase B none, or both A and B comprise a reactant, a catalyst can be present in either phase A or B, and a reactant may in itself form the liquid of phase A or phase B. Thus, it is clear that the concentration of all components of phase A and B can vary in wide ranges. Phase A may comprise:
0-100 wt% liquid
0-100 wt% reactant
0-100 wt% catalyst
And phase B may comprise:
0-100 wt% liquid
0-100 wt% reactant
0-100 wt%

The weight percentages all being the percentage of the total weight of phase A or the phase B, respectively. But within all possible variations, either phase A or B must comprise a reactant, and both phase A and B must be present. If the reactant is also the liquid, the amount of reactant is indicated as wt% reactant and the wt% liquid is said to be 0.

If in the process according to the invention a catalyst is used, the catalyst preferably after phase separation is present in the phase where the products are not predominantly present. This allows easy recirculation of the catalyst containing phase. If in the process according to the invention more than one product are formed, at least one of the products is preferably present predominantly in one phase, while the other product or products are present in the other.phase. This allows for easy recovery of the desired product.

### Description of the figures

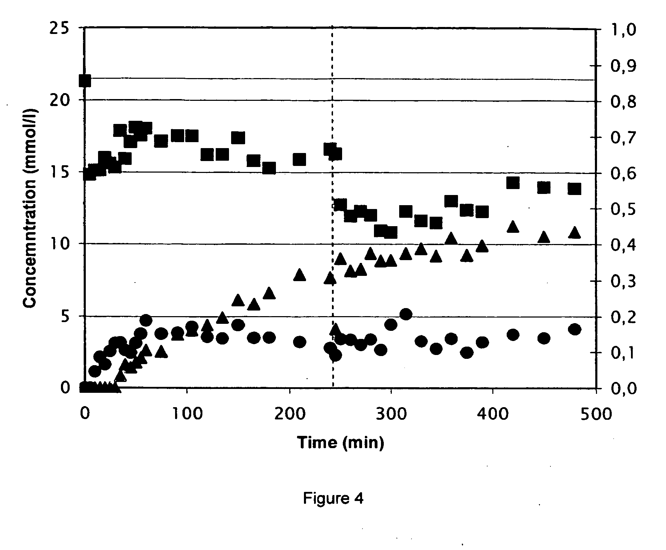

Fig. 1/5 Cross-section of a centrifugal contact-separating device
   I. Mixing zone
   II. Separation zone
   III. Centrifugal contact-separator
Fig. 2/5 The experimental setup used in Example 1
   A. Heptane
   B. Organic feed solution
   C. Reverse osmosis water
   D. Aqueous feed solution
   E. Aqueous waste
   F. CINC V-02 centrifugal contact separator
   G. Organic product
   H. Recycle buffer vessel
   1, 2 and 3 three-way valves
   4 and 5 valves
Fig. 3/5 The experimental setup used in Example 2
   A. Heptane
   B. Organic feed solution
   C. Reverse osmosis water
   D. Aqueous feed solution
   E. Aqueous waste
   F. CINC V-02 centrifugal contact separator
   G. Organic product
   H. Recycle buffer vessel
   K. Organic recycle buffer vessel
   1, 2, 3, 8 and 9 are two-way valves
   4, 5, 6 and 7 are valves
   P1 and P2 are pumps
Fig. 4/5 Gas chromatographic analysis of the organic phase during the ring opening of 1,2-epoxybutane in the CINC V-20 centrifugal contact separator. The initial concentration of the epoxide is indicated by a horizontal line (at ca. 22 mmol/l). After 240 min the substrate solution was recycled (dashed vertical line). The following symbols are used in this figure:
   ■: epoxybutane;
   ▲: nitrobutanol;
   ●: butanediol.
Fig. 5/5 Stability of the haloalcohol dehalogenase enzyme during the centrifugal contact separator experiment

### Examples

### Example 1.

### Enzymatic two-phase catalysis in a centrifugal contact separator

For this and the following experiments use was made of the CIT V-02 (Formerly known as the CINC V-02 for a full description see for instance http://www.auxill.nl/uk/cit.separators.php). Other comparable equipment that can be used is the BXP centrifugal separator from Rousselet-Robatel (http://www.rousselet-robatel.com/products/bxps.php).

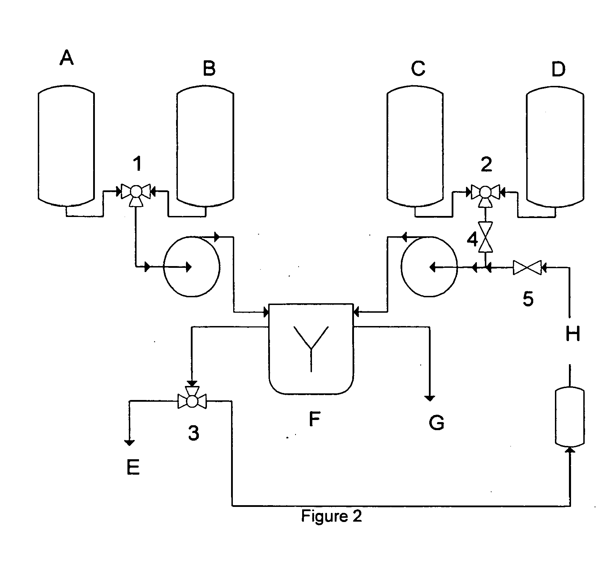

In this example we have used the set-up represented in Figure 2/5.

The aqueous feed solution contained an aqueous buffered solution of the lipase from Rhizomucor miehei, which was prepared by dissolving 155ml of lipase solution (Sigma, ≥ 20 000U/g) in 95 ml of phosphate buffer pH 5.6, prepared by dissolving 26,24 g (192,81 mmol) of KH₂PO₄ and 2,58 g (7,20 mmol) Na₂HPO₄·12 H₂O in 2 L of reverse osmosis water.

The organic feed solution contained 598,15 g (2, 12 mol) of oleic acid, 202,15 g (2,73 mmol) of 1-butanol and 2076 ml of heptane.

The experiments were performed in a CIT V-02 separator. In all cases, the low mix bottom plate was applied. The lay-out of the system is shown in Figure 1. VL1000 control peristaltic tube pumps equipped with a double pump head (1,6 * 1,6 * 8R) were used to feed the CINC reactor with, respectively. The reactions were performed at room temperature. Due to the power input in the system, the reactor temperature increased to about 30°C over a period of 1 h.

The CINC reactor was fed with pure heptane and pure water, both with a flow rate of 6 ml/min. Subsequently, the centrifuge was started (40 Hz, which corresponds to 2380 rpm) and the set-up was allowed to equilibrate for a period of 1 h. At this point, the heptane feed stream was replaced by the organic feed stream (oleic acid/1-butanol in heptane) using three-way valve 1. After equilibration for 10 minutes, the reaction in the CINC was started by replacing the water stream with the aqueous feed stream (aqueous buffer with lipase) using three-way valve 2. For 15 minutes, the CINC reactor was operated once through with respect to both feed streams. In this stage, both the organic and aqueous product streams were discarded. After this stage, three-way valve 3 was turned to collect the aqueous product stream in the recycle buffer vessel. When a quantity of 20 ml has been collected in this buffer vessel, the mode of operation of the aqueous stream was changed from once through to the recycle mode by opening valve 5 and closing valve 4. The reaction was allowed to proceed for 6 h. The oleic acid conversion was determined by measuring the concentration of butyl oleate in the organic stream using gas chromatography. The sample frequency was 5 minutes in the first hour, 15 minutes during the second and third hour and 30 minutes for the remaining 3 hours.

A maximum conversion of 95% of oleic acid after 50 minutes was observed, which leveled off to a constant value of 45% for the remaining reaction time.

### Example 2

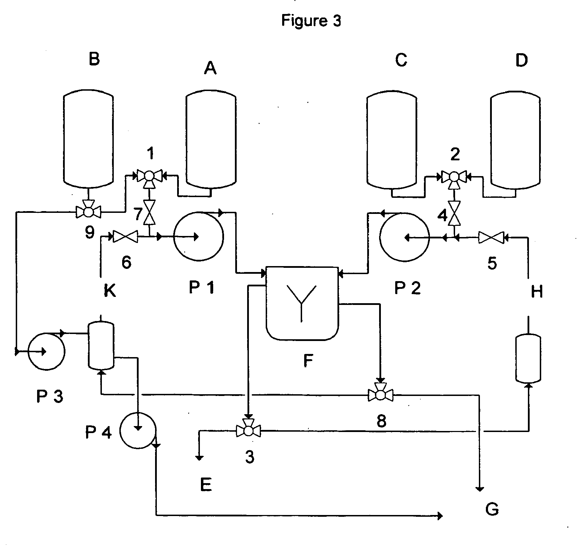

In this example the organic phase is recycled to obtain maximum conversion. The set-up represented in Figure 3/5 was used.

The organic feed stream consisted of 99.99 g (0.35 mol) of oleic acid, 34.71 g (0.47 mmol) of 1-butanol and 350 ml of heptane.

The aqueous feed consisted of 124 ml of lipase solution in 82 ml of phosphate buffer solution (pH=5.6).

The CINC reactor was fed with pure heptane and pure water, both with a flow rate of 6 ml/min. Subsequently, the centrifuge was started (40 Hz, which corresponds to 2380 rpm) and the set-up was allowed to equilibrate for a period of 1 h. At this point, the heptane feed stream was replaced by the organic feed stream (oleic acid, 1-butanol in heptane) using three way valve 1. After equilibration for 10 minutes, the reaction in the CINC was started by replacing the water stream with the aqueous feed stream (aqueous buffer with lipase) using three-way valve 2. For 15 minutes, the CINC reactor was operated once through with respect to both feed streams. In this stage, both the organic and aqueous product streams were discarded. After this stage, three-way valve 3 was switched to collect the aqueous product stream in the aqueous recycle buffer vessel. When a quantity of 20 ml has been collected in this buffer vessel, the mode of operation of the aqueous stream was changed from once through to the recycle mode by opening valve 5 and closing valve 4.

At the same time, three-way valve 8 was turned to collect the organic product stream in the organic recycle buffer vessel. When a quantity of 20 ml has been collected in this buffer vessel, the mode of operation was changed to recycle mode by opening valve 6 and closing valve 7. The feed for pump P 3 was set to the organic feed solution by using three-way valve 9. By switching on pumps P 3 and P 4 (set to a flow rate of 0,6 ml/min) the mode of operation of the organic stream was changed from recycle mode to the partial recycle mode using a 90% recycle. The reaction was allowed to proceed for 4 h. Samples were taken from the organic stream exiting the CINC reactor. The oleic acid conversion was determined by measuring the concentration of butyloleate in the organic stream using gas chromatography. The sample frequency was 5 minutes in the first hour, 15 minutes during the second and third hour and 30 minutes for the remaining hour.

After reaching steady state, which is achieved after about 2 hours of operation, an average conversion of 88% of oleic acid was obtained.

### Example 3

The following reaction was carried out:

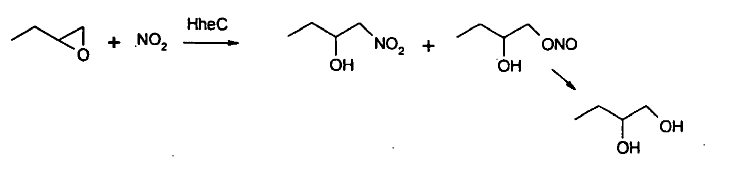

The aqueous phase consisted of 0.2 M phosphate buffer, pH 6.5, containing 1 mM DTT, 307 mM NaNO₂, and 0.5 mg/ml purified haloalcohol dehalogenase (HheC). Final volume: 200 mL

The organic phase was a 24 mM solution of distilled 1,2-epoxybutane in heptane (volume 2.5 L).

Both phases were pumped in the CINC V-2 with a flow rate of 10 ml/min. The enzyme solution was fed for 15 min and then recycled, while the organic phase was continuously streamed for the first 4 hours of experiment and then reused. To follow the reaction course, samples were taken both from the aqueous and organic phase and analyzed by gas chromatography. The results are depicted in Figure 4/5.

Figure 5/5 shows the stability of the Haloalcohol dehalogenase enzyme during the CINC experiment.

It is clear from these results that an enzyme used in a centrifugal contact separator can maintain a high activity over time.

### Example 4 Continuous process for the hydrolysis of butyl formate

In advance of the experiments, a NaOH-solution (23%wt) was bubbled with N₂ for 10 h and the n-Butyl Formate (nBF) for 4 h. One hour before start-up of the experiment, the flow of the water cooling the CINC V-02 (we used a custom made machine which has been provided with a cooling system) was set at 1.1 L/min and the temperature monitored. Before starting to feed the reactants to the reactor, the complete system atmosphere was inerted by alternated vacuum pulling and nitrogen flushing three times. The system was operated under a nitrogen atmosphere.

The experiment was started by starting the engine (50Hz) and the pump feeding the aqueous NaOH solution to the reactor (30 mUmin). The organic phase pump, feeding pure nBF (30 mL/min), was started after the aqueous phase outlet of the reactor started flowing. Samples were taken every two minutes from both phases as soon as the respective outlet started flowing. Twenty minutes after the organic phase outlet started flowing, the experiment was stopped by switching off the pumps and the engine and cleaning the reactor.

The aqueous phase samples were analyzed by titration with 0.1 M hydrochloric acid (using a Contiburette µ10 from CAT Ingenieurbüro M. Zipperer GmbH, Staufen, Germany and a C14 pH-meter from Biochrom Ltd, Cambridge, UK driven by CoachLab II software and computer interface from CMA, Amsterdam, The Netherlands). The organic phase samples were analyzed by GC-FID (HP 5890 series II plus equipped with an HP5 column, injection temperature was 250°C and column temperature 60°C).

Steady-state conversion was determined at 8.4%.

It is clear that by varying the rate of the feeds and the temperature the conversion may be increased to 100% if desired.

### Example 5. Possible chemocatalytic reaction in a centrifugal contact-separator

A chemocatalytic reaction may be carried out in the following way: A catalyst solution is made up from 40 g of Na₂WO₄.2H₂O and 6.6 g of H₂NCH₂PO₃H₂ in 400 ml of water. This solution can be pumped through a centrifugal contact separator in a closed loop. The oxidant, for instance 30% H₂O₂ is charged into the aqueous flow just before entering the centrifugal contact separator. The organic flow may consist of an alkene such as 1-octene containing 1 mol% of methyltri-n-octylammonium hydrogensulfate. Depending on the reactivity of the olefin the organic solution may be preheated from 30-90 degrees C. Flow rates and rotation speed may be adjusted to achieve full conversion. However, it may be advantageous to run oxidation reactions at lower conversion to achieve higher selectivities. The organic stream coming out of the centrifugal contact separator may be washed in a second centrifugal contact separator with a saturated Na₂S₂O₃ solution to remove traces of peroxide. Hereafter the epoxide may be purified by distillation and the remaining olefin fed back to the centrifugal contact separator.

### Example 6. Possible enzymatic kinetic resolution of the ester of a racemic carboxylic acid

The following gives a description of a possible manner in which a centrifugal contact-separator may be used for the enzymatic kinetic resolution of an ester of a racemic carboxylic acid. A catalyst solution is prepared by the addition of 45 g of a lipase (for instance Candida cylindracea lipase(CCL), porcine pancreatic lipase (PPL), Pseudomonas SP lipase (PSL), Candida Antarctica Lipase A (CAL-A), Candida Antarctica Lipase B (CAL-B), to 500 ml of 50-100mM potassium phosphate buffer pH7.0 (50 mg of dry weight per ml of buffer). This buffered suspension is pumped through the centrifugal contact-separator in a closed loop. The organic phase contains an ester, such as ethyl 2-hydroxy-4-phenylbutyrate which is dissolved in heptane (1:1 v/v). The organic solution is also pumped into the centrifugal contact separatorand the organic phase is collected at the upper exit. By regulating the temperature, the flow rates and the concentration of the enzyme it is possible to run the reaction at exactly the desired conversion, usually between 50-60%, depending on the E-factor of the enzyme. The organic exit phase contains the unconverted ester, in this case ethyl (R)-2-hydroxybutyrate, which is the desired product. The aqueous phase becomes enriched in the carboxylate of the other enatiomer (S)-hydroxybutyrate. This can also be isolated in a continuous mode by using a membrane separation if desired. In this latter case the retentate containing the enzyme is sent back to the centrifugal contact separator.

## Claims

1. Use of a centrifugal contact-separator for carrying out a non-radioactive reaction in a liquid-liquid emulsion formed from two immiscible liquids.

2. Use of a centrifugal contact-separator according to claim 1, wherein the reaction is carried out between at least two reactants.

3. Use of a centrifugal contact-separator according to claim 1 or 2, wherein the reaction is a catalytic reaction.

4. Use of a centrifugal contact-separator according to claim 3, wherein a homogeneous catalyst is used.

5. Use of a centrifugal contact-separator according to claim 3, wherein the homogeneous catalyst is an enzyme.

6. Use of a centrifugal contact-separator according to claim 3, wherein the homogeneous catalyst is a transition metal catalyst.

7. Use of a centrifugal contact separator according to any one of claims 1-6 for carrying out a kinetic resolution.

8. A continuous process for carrying out a reaction, comprising the steps of:
i) continuously introducing a liquid phase A and a liquid phase B into at least one first centrifugal contact separator, wherein liquid phases A and B are immiscible and wherein phase A and/or phase B comprise at least one reactant
ii) mixing phase A and phase B thereby allowing an emulsion to be formed
iii) applying a centrifugal force that allows phase separation of the emulsion, such that phases A' and B' are obtained;
iv) optionally, recovering a reaction product from at least one of the phases A' and B'.

9. A process according to claim 8, wherein phase A comprises a reactant and phase B comprises a different reactant.

10. A process according to claim 8 or 9 wherein a catalyst is present.

11. A process according to any one of claims 8-10, wherein the catalyst after phase separation of the emulsion is predominantly present in either phase A' or phase B'.

12. A process according to claim 11 wherein at least 90 % of the total amount of catalyst present in phases A' and B' after phase separation of the emulsion is present in either phase A' or phase B'.

13. A process according to claim 11 or 12 wherein the phase predominantly comprising the catalyst is recycled into the contact separator through the inlet for phase A.

## Patentansprüche

1. Verwendung eines Zentrifugalkontaktseparators zur Durchführung einer nichtradioaktiven Reaktion in einer aus zwei nicht mischbaren Flüssigkeiten gebildeten Flüssig-Flüssig-Emulsion.

2. Verwendung eines Zentrifugalkontaktseparators nach Anspruch 1, bei der die Reaktion zwischen mindestens zwei Reaktanten durchgeführt wird.

3. Verwendung eines Zentrifugalkontaktseparators nach Anspruch 1 oder 2, bei der es sich bei der Reaktion um eine katalytische Reaktion handelt.

4. Verwendung eines Zentrifugalkontaktseparators nach Anspruch 3, bei der ein Homogenkatalysator verwendet wird.

5. Verwendung eines Zentrifugalkontaktseparators nach Anspruch 3, bei der es sich bei dem Homogenkatalysator um ein Enzym handelt.

6. Verwendung eines Zentrifugalkontaktseparators nach Anspruch 3, bei der es sich bei dem Homogenkatalysator um einen Übergangsmetallkatalysator handelt.

7. Verwendung eines Zentrifugalkontaktseparators nach einem der Ansprüche 1 bis 6 zur Durchführung einer kinetischen Racemattrennung.

8. Kontinuierliches Verfahren zur Durchführung einer Reaktion, bei dem man:
i) eine flüssige Phase A und eine flüssige Phase B kontinuierlich in mindenstens einen ersten Zentrifugalkontaktseparator einträgt, wobei die flüssigen Phasen A und B nicht mischbar sind und Phase A und/oder Phase B mindestens einen Reaktanten enthält;
ii) Phase A und Phase B mischt, wodurch sich eine Emulsion bilden kann;
iii) eine Zentrifugalkraft anlegt, die die Phasentrennung der Emulsion erlaubt, so daß man die Phasen A' und B' erhält;
iv) gegebenenfalls aus mindestens einer der Phasen A' und B' ein Reaktionsprodukt gewinnt.

9. Verfahren nach Anspruch 8, bei dem die Phase A einen Reaktanten enthält und die Phase B einen anderen Reaktanten enthält.

10. Verfahren nach Anspruch 8 oder 9, bei dem ein Katalysator zugegen ist.

11. Verfahren nach einem der Ansprüche 8-10, bei dem der Katalysator nach der Phasentrennung der Emulsion hauptsächlich in Phase A' oder Phase B' vorliegt.

12. Verfahren nach Anspruch 11, bei dem mindestens 90% der Gesamtmenge an Katalysator in den Phasen A' und B' nach der Phasentrennung der Emulsion in Phase A' oder Phase B' vorliegen.

13. Verfahren nach Anspruch 11 oder 12, bei dem die hauptsächlich den Katalysator enthaltende Phase durch den Einlaß für Phase A in den Kontaktseparator zurückgeführt wird.

## Revendications

1. Utilisation d'un séparateur à contact centrifuge pour conduire une réaction non radioactive dans une émulsion liquide-liquide formée de deux liquides immiscibles.

2. Utilisation d'un séparateur à contact centrifuge selon la revendication 1, **caractérisée en ce que** la réaction est conduite entre au moins deux réactifs.

3. Utilisation d'un séparateur à contact centrifuge selon la revendication 1 ou 2, **caractérisée en ce que** la réaction est une réaction catalytique.

4. Utilisation d'un séparateur à contact centrifuge selon la revendication 3, **caractérisée en ce qu'**un catalyseur homogène est utilisé.

5. Utilisation d'un séparateur à contact centrifuge selon la revendication 3, **caractérisée en ce que** le catalyseur homogène est une enzyme.

6. Utilisation d'un séparateur à contact centrifuge selon la revendication 3, **caractérisée en ce que** le catalyseur homogène est un catalyseur à métal de transition.

7. Utilisation d'un séparateur à contact centrifuge selon l'une quelconque des revendications 1 à 6 pour conduire une résolution cinétique.

8. Procédé continu pour conduire une réaction, comprenant les étapes consistant à :
i) introduire en continu une phase liquide A et une phase liquide B dans au moins un premier séparateur à contact centrifuge, où les phases liquides A et B sont immiscibles et où la phase A et/ou la phase B comprennent au moins un réactif,
ii) mélanger la phase A et la phase B de manière à permettre qu'une émulsion soit formée,
iii) appliquer une force centrifuge qui permet la séparation de phases de l'émulsion, de sorte que des phases A' et B' soient obtenues ;
iv) facultativement, récupérer un produit de réaction à partir d'au moins une des phases A' et B'.

9. Procédé selon la revendication 8, **caractérisé en ce que** la phase A comprend un réactif et la phase B comprend un réactif différent.

10. Procédé selon la revendication 8 ou 9 **caractérisé en ce qu'**un catalyseur est présent.

11. Procédé selon l'une quelconque des revendications 8 à 10, **caractérisé en ce que** le catalyseur après séparation de phases de l'émulsion est principalement présent dans la phase A' ou la phase B'.

12. Procédé selon la revendication 11 **caractérisé en ce qu'**au moins 90 % de la quantité totale de catalyseur présent dans les phases A' et B' après séparation de phases de l'émulsion est présent dans la phase A' ou la phase B'.

13. Procédé selon la revendication 11 ou 12 **caractérisé en ce que** la phase comprenant principalement le catalyseur est recyclée dans le séparateur à contact par l'intermédiaire de l'entrée de la phase A.
